# EUROPÄISCHE PATENTSCHRIFT

(11) **EP 0 450 438 B1**
(45) Veröffentlichungstag und Bekanntmachung des Hinweises auf die Patenterteilung: **20.07.1994**
(21) Anmeldenummer: 91104621.7
(22) Anmeldetag: 23.03.1991
(51) Int. Cl.: C07D 277/40

(54) **Verfahren zur Herstellung von 2-Amino-5-formyl-4-halogenthiazolen**
Process for the production of 2-amino-5-formyl-4-halogen thiazoles
Procédé pour la production de 2-amino-5-formyl-4-halogène thiazoles

(30) Priorität: 02.04.1990 DE 4010514
(43) Veröffentlichungstag der Anmeldung: 09.10.1991
(73) Patentinhaber: BASF Aktiengesellschaft, 67063 Ludwigshafen (DE)
(72) Erfinder: Wiesenfeldt, Matthias, Dr., W-6704 Mutterstadt (DE); Etzbach, Karl-Heinz, Dr., W-6710 Frankenthal (DE)

(56) Entgegenhaltungen:
- DE-C- 3 108 077

## Beschreibung

Die vorliegende Erfindung betrifft ein neues Verfahren zur Herstellung von 2-Amino-5-formyl-4-halogenthiazolen durch Umsetzung zunächst von Thioharnstoff mit Chlor- oder Bromessigsäure in Gegenwart eines N,N-disubstituierten Formamids und Behandlung des Reaktionsgemisches mit einem Phosphorsäuretrihalogenid und anschließend mit Wasser in Gegenwart einer Base.

2-Amino-5-formyl-4-halogenthiazole sind aus der DE-A-3 015 121 bekannt. Sie werden dort durch Umsetzung von 2-Aminothiazolin-4-onen mit einem Vilsmeier-Reagens erhalten. Nachteilig bei diesem Verfahren ist die Tatsache, daß zunächst ein Zwischenprodukt aus der Thiazolinreihe hergestellt und isoliert werden muß, an dem dann die Vilsmeier-Reaktion vorgenommen wird.

Die Herstellung des Zwischenproduktes 2-Aminothiazolin-4-on aus Chloressigsäure und Thioharnstoff ist beispielsweise in Ukr. Khim. Zh., Band 27, Seiten 680 und 681, 1961, oder in Chem. Abstr., Band 103, 104 522 h, 1985, beschrieben. Als Reaktionsmedium dient dort Isobutanol oder Wasser.

Aufgabe der vorliegenden Erfindung war es deshalb, ein neues Verfahren zur Herstellung von 2-Amino-5-formyl-4-halogenthiazolen bereitzustellen, bei dem auf die Anwendung von heterocyclischen Zwischenprodukten verzichtet werden kann und das die Zielprodukte in guter Ausbeute und hoher Reinheit liefert.

Es wurde nun gefunden, daß die Herstellung von 2-Amino-5-formyl-4-(chlor- oder brom)thiazol vorteilhaft gelingt, wenn man
a) in einem 1. Schritt Thioharnstoff mit Chlor- oder Bromessigsäure in Gegenwart eines N,N-Di(C₁-C₄-alkyl)formamids oder eines N-(C₁-C₄-Alkyl)-N-phenylformamids umsetzt und das Reaktionsgemisch
b) in einem 2. Schritt mit Phosphoroxidtrichlorid oder Phosphoroxidtribromid und anschließend
c) in einem 3. Schritt mit Wasser in Gegenwart einer Base behandelt.

Bevorzugt wird das neue Verfahren zur Herstellung von 2-Amino-5-formyl-4-chlorthiazol angewandt.

Geeignete substituierte Formamide, die im 1. Schritt des Verfahrens zur Anwendung kommen können, sind z.B. N,N-Dimethylformamid, N,N-Diethylformamid oder N-Methyl-N-phenylformamid. Die Verwendung von N,N-Dimethylformamid ist bevorzugt.

Das erfindungsgemäße Verfahren wird zweckmäßig so durchgeführt, daß man zunächst Thioharnstoff und substituiertes Formamid vorlegt und zu dieser Mischung bei einer Temperatur von 0 bis 30°C Chloressigsäure oder Bromessigsäure zugibt. Das Molverhältnis Thioharnstoff:Chloressigsäure oder Bromessigsäure beträgt in der Regel 1:1 bis 1:1,5. Das Molverhältnis Thioharnstoff:substituiertes Formamid beträgt im allgemeinen 1:5 bis 1:30. Die Umsetzung wird bei einer Temperatur von 20 bis 100°C, vorzugsweise 50 bis 90°C und insbesondere 60 bis 85°C, vorgenommen.

Es ist auch möglich, den 1. Schritt in Anwesenheit eines zusätzlichen inerten organischen Verdünnungsmittels durchzuführen. Geeignete inertere Verdünnungsmittel sind z.B. Benzoesäure-C₁-C₄-Alkylester, wie Benzoesäuremethylester oder Benzoesäureethylester, Chlorbenzol oder Nitrobenzol.

Nach einer Reaktionsdauer, die üblicherweise 1 bis 3 Stunden in Anspruch nimmt, wird das Reaktionsgemisch im 2. Schritt bei einer Temperatur von 0 bis 40°C mit Phosphoroxidtrichlorid oder Phosphoroxidtribromid versetzt und danach bei einer Temperatur von 50 bis 110°C, vorzugsweise 60 bis 90°C, umgesetzt.

Die Verwendung von Phosphoroxidtrichlorid ist bevorzugt.

Je Mol Thioharnstoff (1. Schritt) werden im allgemeinen 1 bis 5 Mol, vorzugsweise 2 bis 4 Mol und insbesondere ca. 3,5 Mol, an Phosphoroxidtrichlorid oder Phosphoroxidtribromid zugegeben.

Nach Zugabe des Phosphoroxidtrihalogenids wird in der Regel 3 bis 7 Stunden bei der obengenannten Temperatur nachgerührt. Dann wird das Reaktionsgemisch im 3. Schritt mit Wasser in Gegenwart einer Base behandelt.

Dazu kann das Reaktionsgemisch, das in der Regel eine Temperatur von 30 bis 100°C, vorzugsweise 40 bis 80°C, aufweist, zu einer wäßrigen Lösung einer Base gegeben werden. Um eine günstige Temperaturführung zu erreichen, ist es aber von besonderem Vorteil, das Reaktionsgemisch zu einer Mischung aus Base und Eis zu geben.

Geeignete Basen sind z.B. Alkali- oder Erdalkalihydroxide, wie Natriumhydroxid, Kaliumhydroxid, Magnesiumhydroxid oder Calciumhydroxid, Pyridin oder Amine, wie Triethylamin, Tripropylamin, Triisopropylamin, Tributylamin oder N,N-Diethylanilin. Die Verwendung von Alkalihydroxiden oder Erdalkalihydroxiden, insbesondere von Calciumhydroxid, ist bevorzugt.

Je Mol Thioharnstoff (1. Schritt) werden im allgemeinen 3 bis 15 Mol, vorzugsweise 4 bis 8 Mol, an Base verwendet. Die Menge an Base, bezogen auf Wasser oder Eis, beträgt üblicherweise 2 bis 12 Gew.-%.

Nach beendeter Umsetzung, die in der Regel 1 bis 5 Stunden in Anspruch nimmt, wird das Zielprodukt mittels Natriumchlorid bei einer Temperatur von 0 bis 30°C ausgesalzen. Das Natriumchlorid kann dabei bereits zu Beginn des 3. Schritts zur wäßrigen Lösung der Base oder zur Mischung aus Base und Eis hinzugefügt werden. Das als Niederschlag anfallende Zielprodukt wird dann abgetrennt, gewaschen und getrocknet.

Mittels des erfindungsgemäßen Verfahrens, das sowohl in kontinuierlicher als auch in diskontinuierlicher Arbeitsweise vorgenommen werden kann, gelingt es, 2-Amino-4-formyl-5-(chlor- oder -brom)thiazol in einfacher Weise in hoher Reinheit und guter Ausbeute herzustellen. Der besondere Vorteil des neuen Verfahrens liegt darin, daß die gesamte Umsetzung, die aus mehreren Reaktionsschritten besteht, ohne Isolierung der jeweils auftretenden Zwischenprodukte vorgenommen werden kann, wobei die Schritte 1 und 2 direkt als Eintopfsynthese durchgeführt werden können. Daraus resultiert eine höhere Raum-Zeit-Ausbeute.

Bei 2-Amino-5-formyl-4(chlor- oder -brom)thiazol handelt es sich um wertvolle Zwischenprodukte zur Synthese von Azofarbstoffen, wobei sie gemäß der DE-A-3 108 077 als Diazokomponente verwendet werden können.

Das folgende Beispiel soll die Erfindung näher erläutern.

### Beispiel

In einem Kolben wurden 152 g Thioharnstoff und 1,5 1 N,N-Dimethylformamid vorgelegt und bei Raumtemperatur unter Rühren 190 g Chloressigsäure zugegeben. Das Reaktionsgemisch wurde bei 75 bis 80°C 2 Stunden nachgerührt und anschließend auf 40°C abgekühlt. Bei dieser Temperatur wurden 644 ml Phoshoroxidtrichlorid eingetropft. Das Gemisch wurde 30 Minuten bei 60°C nachgerührt und anschließend auf 90°C erhitzt und 5 Stunden bei dieser Temperatur gerührt. Danach wurde das Reaktionsgemisch in eine Mischung von 683 g Natriumchlorid und 504 g Calciumhydroxid in 5 kg Eis gerührt.

Nach dem Abklingen der exothermen Reaktion wurde nochmals 1 1 Wasser zugesetzt und das Gemisch dann 3 bis 4 Stunden bei 55°C gerührt. Danach wurde die Lösung auf 0°C abgekühlt, der resultierende Niederschlag abgesaugt, mit Wasser neutral gewaschen und unter vermindertem Druck getrocknet. Man erhielt 246,7 g (76 % d.Th.) 2-Amino-5-formyl-4-chlorthiazol.

## Patentansprüche

1. Verfahren zur Herstellung von 2-Amino-5-formyl-4-(chlor oder -brom)thiazol, dadurch gekennzeichnet, daß man
a) in einem 1. Schritt Thioharnstoff mit Chlor- oder Bromessigsäure in Gegenwart eines N,N-Di(C₁-C₄-alkyl)formamids oder eines N-(C₁-C₄-Alkyl)-N-phenylformamids umsetzt und das Reaktionsgemisch
b) in einem 2. Schritt mit Phosphoroxidtrichlorid oder Phosphoroxidtribromid und anschließend
c) in einem 3. Schritt mit Wasser in Gegenwart einer Base behandelt.

2. Verfahren nach Anspruch 1, dadurch gekennzeichnet, daß man die Behandlung im 2. Schritt mit Phosphoroxidtrichlorid vornimmt.

## Claims

1. A process for preparing 2-amino-5-formyl-4-(chloro or bromo)thiazole comprising
a) a first step of reacting thiourea with chloroacetic or bromoacetic acid in the presence of an N,N-di(C₁-C₄-alkyl)-formamide or of an N-(C₁-C₄-alkyl)-N-phenylformamide,
b) a second step of treating the reaction mixture with phosphorus oxychloride or oxybromide, and
c) a 3rd step of subsequently treating the reaction mixture with water in the presence of a base.

2. A process as claimed in claim 1, wherein the treatment of step 2 is carried out with phosphorus oxychloride.

## Revendications

1. Procédé de préparation de 2-amino-5-formyl-4- (chloro ou bromo)thiazole, caractérisé en ce que
a) dans une 1ème étape, on fait réagir de la thiourée avec de l'acide chloro- ou bromoacétique en présence d'un N,N-di(alkyl en C₁-C₄)formamide ou d'un N-(alkyl en C₁-C₄)-N-phénylformamide et
b) dans une 2ème étape, on traite le mélange réactionnel par de l'oxytrichlorure de phosphore ou de l'oxytribromure de phosphore et ensuite
c) dans une 3ème étape, on le traite par de l'eau en présence d'une base.

2. Procédé selon la revendication 1, caractérisé en ce qu'on effectue le traitement dans la 2ème étape avec de l'oxytrichlorure de phosphore.
